# EUROPÄISCHE PATENTSCHRIFT

(11) **EP 2 852 824 B1**
(45) Veröffentlichungstag und Bekanntmachung des Hinweises auf die Patenterteilung: **11.03.2020**
(21) Anmeldenummer: 13723817.6
(22) Anmeldetag: 22.05.2013
(51) Int. Cl.: A61F 13/00, A61F 13/02, A61F 13/08, B65H 18/10, B65H 18/26, G01N 3/08, G01N 33/36

(54) **MESSVORRICHTUNG ZUM BESTIMMEN DES KOMPRESSIONSDRUCKS VON KOMPRESSIONSBINDEN**
MEASURING DEVICE FOR DETERMINING THE COMPRESSIVE PRESSURE OF COMPRESSION DRESSINGS
DISPOSITIF DE MESURE PERMETTANT DE DÉTERMINER LA PRESSION DE COMPRESSION DE BANDAGES COMPRESSIFS

(30) Priorität: 23.05.2012 DE 102012208640
(43) Veröffentlichungstag der Anmeldung: 01.04.2015
(73) Patentinhaber: Karl Otto Braun GmbH & Co. KG., 67752 Wolfstein (DE)
(72) Erfinder: KLOEPPELS, Michael, 52249 Eschweiler (DE); HARRER, Heinrich, 67697 Otterberg (DE)
(74) Vertreter: DREISS Patentanwälte PartG mbB
(86) Internationale Anmeldenummer: PCT/EP2013/060494
(87) Internationale Veröffentlichungsnummer: WO 2013/174860

(56) Entgegenhaltungen:
- DE-A1- 3 829 199
- DE-A1- 4 430 566
- DE-B- 1 124 268
- GB-A- 2 473 321

## Beschreibung

Die Erfindung betrifft eine Messvorrichtung zum Bestimmen des Kompressionsdrucks von therapeutischen Kompressionsmaßnahmen, nämlich Kompressionsbinden.

Kompressionsmaßnahmen sind beispielsweise sogenannte Kompressionsstrümpfe oder Kompressionsstrumpfhosen, allgemein Strümpfe oder Strumpfhosen mit einer gewissen Stützwirkung, aber auch entsprechende, zumeist schlauchförmige Gebilde für den Armbereich, wobei derartige Maßnahmen im Armbereich zumeist nach Brustoperationen eingesetzt werden, um Flüssigkeitsansammlungen in den Armen zu verhindern bzw. zu beheben. Bei den erfindungsgemäß verwendeten Kompressionsmaßnahmen handelt es sich um Kompressionsbinden und -bandagen, die hier stets als Kompressionsbinden bezeichnet werden sollen. Diese liegen in Form von Flachmaterialien vor, die zum Aufbringen einer Kompression an der gewünschten Gliedmaße angebracht werden, d. h. auf diese aufgewickelt werden. Derartige Kompressionsbinden bestehen zumeist aus Geweben, Gewirken oder übernähten Vliesen.

Gängige Indikationen für eine Kompressionstherapie können alle Arten von chronischer venöser Insuffizienz, in den verschiedenen Stadien bis hin zu offenen Beinen (venöse Beinulcera), aber auch Schwellungen jeglicher Art, wie Lymphödeme, Lipodeme, tiefe Venenthrombose, Thrombophelitis, Varikosis und postchirurgische Verbände, z.B. nach Varizen oder Skleroisierung sein.

Für den Erfolg der Therapie von entscheidender Bedeutung ist, dass ausreichende, aber nicht zu hohe Drücke an der Körperoberfläche erzielt werden. Die Höhe des Kompressionsdrucks hängt insbesondere von der Eigenschaft des Materials, der Verarbeitung bzw. Fertigungstechnik und Anlegetechnik ab.

Die Aufnahme von Parametern, insbesondere der aufgebrachten Kompression bzw. des Drucks, ist zum einen für die Herstellung bzw. Entwicklung entsprechender Kompressionsbinden von Vorteil, kann zum anderen jedoch auch zum Training von Patienten, bei denen entsprechende Kompressionsmaßnahmen eingesetzt werden sollen, bzw. zum Training von medizinischem und therapeutischem Personal verwendet werden.

Es sind im Stand der Technik bereits Systeme mit starren Grundkörpern in Form von zumeist Gliedmaßennachbildungen bekannt. So sei beispielsweise auf die US-PS 6,334,363 B1 verwiesen, die eine Vorrichtung zum Messen von Druckpunkten durch therapeutische Kompressionsmaßnahmen beschreibt, wobei eine Vielzahl von Sensoren vorgesehen sind, die auf der Oberfläche der starren, einem Bein entsprechenden Form angebracht sind. Durch die gleichzeitige Messung der Drücke an allen Messpunkten und die Vielzahl der Messpunkte kann ein Druckprofil der Kompressionsmaßnahme erzeugt werden. Dabei kann über die Kraft-Dehnungs-Beziehung eines spezifischen Kompressionsmaterials und die Anwendung der Formel von Laplace theoretisch berechnet werden, welche Punktdrücke in Abhängigkeit des Radius des Untergrunds resultieren, wobei dies ausschließlich für zylindrische und starre Körper gilt.

Der menschliche Unterschenkel, aber auch der menschliche Arm als die Hauptanwendungsgebiete für Kompressionsmaßnahmen sind jedoch weder streng zylindrisch noch starr. Vielmehr entsteht je nach Ausprägung der Muskulatur und der Beweglichkeit des oberen Sprunggelenks bzw. Knies eine Verschiebung der Muskelbäuche bei der Muskelkontraktion, z. B. beim Laufen. Damit entsteht ein dynamisches System, bei dem die Umfänge am Unterschenkel mit jedem Schritt annähernd zyklisch verändert werden. Davon betroffen sind die resultierenden Drücke unter einer Kompressionsmaßnahme, die sich entsprechend mit dem Schrittzyklus ändert. Bei einer am erschlafften Muskel angelegten Binde spricht man vom Ruhe-, bei einer am kontrahierten Muskel angelegten Binde vom Arbeitsdruck. Das Verhältnis von Ruhe- zu Arbeitsdruck einer Kompressionsmaßnahme ist dabei eine der relevanten Größen für die Wirksamkeit einer Therapiemaßnahme.

Zur Verbesserung wurde beispielsweise in der EP 1 118 851 A1 eine Vorrichtung zum Messen der Kompression durch Strumpfwarenartikel beschrieben, bei der ein Unterstrumpf vorgesehen ist aus einzelnen röhrenförmigen Elementen, die zum Teil aus Schalen aufgebaut sind, wobei die Schalen sich aufspreizen lassen, um Stärkevariationen entlang der Länge eines Beins nachbilden zu können. Auf diese Weise können erste elastische Eigenschaften einer Strumpfware aufgenommen werden. Nachteilig ist jedoch hierbei, dass die Nachbildung des Bewegungsapparats aufgrund der einzelnen röhrenförmigen Elemente sehr abstrahiert ist und darüber hinaus durch die mechanische Aufspreizung der röhrenförmigen Segmente ein Simulation eines Bewegungsablaufs nur sehr unvollkommen möglich ist.

Des Weiteren ist aus der EP 1 703 269 A1 eine Einrichtung mit einem länglichen, vertikal verlaufenden Träger für Drucksensoren bekannt, wobei der Träger den Bereich des Schienbeins simuliert sowie Bereiche der Wade bzw. des Oberschenkels des Beins. Der zu messende Strumpf wird über den Träger und ein Gegenlager gestülpt. Dabei sind Drucksensoren vorgesehen sowie ein aufwendiger Hebelmechanismus, der eine Aufspreizung in der Form einer Schere ermöglicht, um so eine Anpassung sowohl an die variable Breite als auch Länge von Beinen zu gewährleisten.

Weitere Messapparate zur Messung von Drücken, die durch Kompressionsmaßnahmen aufgebracht werden, sind aus der US-PS-4,137,763 sowie der GB-A-2 168 156 bekannt.

Dabei existieren grundsätzlich verschiedene Druckgradienten bzw. Druckkennwerte, die ausschlaggebend für die Beurteilung eines Verbandes sein können. Zwei mögliche Druckkennwerte sind:

### DSI (Dynamic Stiffness Index):

Der DSI gibt die Höhe der Amplitude der (oszillierenden) Druckschwankung bei der Dorsiflexion (Wippen des Fußes im Liegen) an. Er wird in mm Hg angegeben.

### SSI (Static Stiffness Index):

Der SSI gibt die Differenz des Drucks im Stehen (unter Belastung) und des Drucks im Liegen (Ruhedruck) an und wird ebenfalls in mm Hg angegeben.

SSI-Werte werden nach bestimmten Vorgaben in vivo an einem Bein gemessen, an dem eine Kompressionsbinde angelegt ist.

Die Messungen des SSI und des DSI sind jedoch in vivo mit möglichen Fehlerquellen und großen Messungenauigkeiten verbunden. So spielen folgende Parameter eine Rolle:
- Beinform/Konfiguration (Dicke, lokale Änderung des Beinumfangs beim Bewegen, ...)
- Gewebekonsistenz des Beins an der Messstelle
- Länge des Beins (Unterschenkel im Verhältnis zur Länge der Kompressionsmaßnahme, insbesondere der Kompressionsbinde)
- Messkopfform, Messkopfdicke
- Messort
- Radius der Beinkrümmung an der Messstelle
- Anwickeldruck, Anwickelart ("Anwickelverhalten")
- Startdruck (Ruhedruck nach Bewicklung zum Start der Messung).

Die genannten Einflussgrößen führen zu einer großen Streuung der Werte und dazu, dass die Vergleichbarkeit der Werte untereinander schwierig ist.

Dabei gibt der Static Stiffness Index bei einer in vivo- bzw. einer in vitro-Messung die Differenz von zwei Drücken an bei einer Umfangsänderung des Beins vom 1 cm, wodurch die Lageänderung (Liegen/Stehen) abgebildet wird.

Ein weiterer Ungenauigkeitseinfluss sowohl bei in vivo- als auch in vitro-Messungen liegt in der Wahl des Messsensors. Größe und Dicke des Sensors haben einen erheblichen Einfluss. So stellt ein punktueller Messkopf auf einem Bein oder einer Röhre, der durch eine Kompressionsbinde mit einer Kompression beaufschlagt wird, einen Störfaktor in der Messung dar, da er eine Auswölbung verursacht. Daneben hat der Krümmungsradius einer Gliedmaße oder des sonstigen Grundkörpers, je nach Messkopfgröße und der Biegung des Messkopfes, einen Einfluss auf das Messergebnis.

So ist beispielsweise aus der WO 2006/012986 A1 ein System zum Die genannten Einflussgrößen führen zu einer großen Streuung der Werte und dazu, dass die Vergleichbarkeit der Werte untereinander schwierig ist.

Dabei gibt der Static Stiffness Index bei einer in vivo- bzw. einer in vitro-Messung die Differenz von zwei Drücken an bei einer Umfangsänderung des Beins vom 1 cm, wodurch die Lageänderung (Liegen/Stehen) abgebildet wird.

Ein weiterer Ungenauigkeitseinfluss sowohl bei in vivo- als auch in vitro-Messungen liegt in der Wahl des Messsensors. Größe und Dicke des Sensors haben einen erheblichen Einfluss. So stellt ein punktueller Messkopf auf einem Bein oder einer Röhre, der durch eine Kompressionsbinde mit einer Kompression beaufschlagt wird, einen Störfaktor in der Messung dar, da er eine Auswölbung verursacht. Daneben hat der Krümmungsradius einer Gliedmaße oder des sonstigen Grundkörpers, je nach Messkopfgröße und der Biegung des Messkopfes, einen Einfluss auf das Messergebnis.

So ist beispielsweise aus der WO 2006/012986 A1 ein System zum Messen des Kompressionsdrucks bekannt, wobei auch hier lediglich punktuell gemessen wird und auch nur eine Messung an der Oberfläche möglich ist. Zwar sieht im Gegensatz zu anderen Messvorrichtungen die Druckschrift eine Simulationseinrichtung für einen Muskel vor, eine gleichmäßige Umfangsänderung, wie sie für die Messung des Static Stiffness Index notwendig und damit eine Anpassung an verschiedene Beindurchmesser und -längen ist, ist jedoch nicht möglich.

Aus der DE 44 30 566 A1 ist ein Verfahren bekannt, bei dem Abweichungen von der geradzylindrischen Kontur einer Fadenspule während des Aufwickelns des Fadens ermittelt werden und z.B. über eine Änderung der Changierung korrigiert werden können.

GB2473321 A offenbart eine Vorrichtung zum Bestimmen des Kompressionsdrucks von Kompressionsbinden umfassend einen um seine Längsachse drehbaren Grundkörper mit Drucksensoren.

Es ist daher Aufgabe der Erfindung, eine Messvorrichtung gemäß Anspruch 1 bereitzustellen für die reproduzierbare und standardisierte in vitro-Messung des Kompressionsdrucks bei Kompressionsbinden, insbesondere des Static Stiffness Index.

Weise eliminiert werden.

Besonders bevorzugt kann der Grundkörper eine zylindrische oder kegelstumpfförmige Grundform aufweisen, wobei der Querschnitt des Grundkörpers rund oder oval ist.

Dabei kann der Grundkörper sowohl hinsichtlich seiner Grundform als auch hinsichtlich seiner Querschnittsform über die Länge variabel sein. Insbesondere kann jedoch auch vorgesehen sein, dass Grundform und auch Querschnittsform erhalten bleiben, jedoch der Querschnitt einstellbar bzw. der Umfang des Grundkörpers einstellbar ist mittels einer Verstellvorrichtung. Auf diese Weise kann eine naturgerechte Nachstellung einer Gliedmaße bei gleichzeitig personenunabhängiger standardisierte Anlegung einer Kompressionsbinde erfolgen.

Der Grundkörper weist dabei eine bevorzugte Länge von 300 bis 700 mm, besonders bevorzugt von 400 bis 600 und besonders bevorzugt von 500 mm auf.

Er kann dabei insbesondere aus Metall, insbesondere aus Aluminium oder auch aus (Hart-)Kunststoff oder Kombinationen hieraus ausgebildet sein.

Der Grundkörper weist dabei bevorzugt einen Durchmesser von 70 bis 90 mm und besonders bevorzugt von 80 mm auf. Sofern der Grundkörper hinsichtlich seines Umfangs oder Durchmessers veränderbar ist, kann vorgesehen sein, dass ein erster geringerer Durchmesser dem oben angegebenen Durchmesser entspricht und ein zweiter größerer Durchmesser zwischen 80 bis 100 mm und vorzugsweise 90 mm beträgt. Insbesondere beträgt der Unterschied zwischen den beiden einstellbaren Umfängen 10mm. Bei der Bestimmung des des SSI muss sich der Umfang um 10mm ändern. Bsp: Durchmesser D1 = 80mm und Umfang1 = 251 mm. Umfang2 = Umfang1 + 10 mm = 251mm + 10 mm = 261 mm. D.h. der Durchmesser D2 = 83,2 mm. Sofern auf dem Grundkörper weitere Schichten angeordnet sind, ist die Umfangsänderung von insbesondere 10 mm auf die äußerste Schicht bezogen, die auf dem Grundkörper angeordnet ist.

Darüber hinaus kann vorgesehen sein, dass ein zum Aufwickeln einer Kompressionsbinde vorgesehener Bereich des Grundkörpers 200 bis 400 mm und besonders bevorzugt 250 bis 350 mm und besonders bevorzugt 300 mm lang ist. Hierdurch wird die durchschnittliche Länge eines menschlichen Beins bestmöglich nachgebildet.

Die Messvorrichtung weist dabei eine Längs- und eine Querrichtung auf, wobei die Längsrichtung der Längsrichtung des Grundkörpers entspricht oder es sich bei der Querrichtung um die radiale Richtung handelt.

Eine derartige Messvorrichtung bietet die Möglichkeit der vergleichenden Messung verschiedener Kompressionsbinden zueinander sowie die vergleichende Messung verschiedener Wickelmaßnahmen. So kann beispielsweise bei Verwendung der gleichen Kompressionsbinde ermittelt werden, wie sich bei Erhöhung des Anlegezugs der Druck an einem Bein verändert bzw. wie beispielsweise die Erhöhung der Überlappung der einzelnen Bindentouren sich in einem variierenden Kompressionsdruck niederschlägt.

Darüber hinaus können auch verschiedene Kompressionsbinden miteinander verglichen werden und insbesondere können Modifikationen an den Kompressionsbinden unmittelbar erfolgen. Schließlich lassen sich auch Veränderungen über die Zeit, insbesondere der Druckverlauf über die Zeit, einer durch eine Kompressionsbinde erfolgten Kompressionsmaßnahme verfolgen und es können Alterungsbedingungen, wie beispielsweise der Zustand nach verschiedenen oder mehreren Waschzyklen, verfolgt werden.

Nach einer ersten Ausgestaltung kann vorgesehen sein, dass der Grundkörper eine zylindrische oder kegelstumpfförmige Grundform aufweist, die sich insbesondere zu einem gedachten Fuß bzw. zu einer gedachten Hand, also nach distal in Längsrichtung, einer Gliedmaße verjüngt. Dabei kann der Querschnitt rund oder oval sein, wobei eine ovale Form die tatsächlichen Gegebenheiten an einer Gliedmaße, insbesondere an einem Bein, besser abbildet. Grundsätzlich ist auch ein eiförmiger Querschnitt oder ein anderer Querschnitt denkbar.

Weiterhin kann vorgesehen sein, dass sowohl die Grundform als auch die Querschnittsform über die Länge des Grundkörpers variabel ist. So kann z. B. eine kegelförmige Grundform in Längsrichtung in eine zylindrische Grundform übergehen oder es kann eine runde Querschnittsform in eine ovale oder eiförmige Form überführt werden. Hierdurch können die Gegebenheiten an einer Gliedmaße noch besser abgebildet werden. Darüber hinaus ist es auch möglich, dass der Grundkörper eine einstellbare Querschnittsform und/oder einen einstellbaren Umfang aufweist. Dies kann z. B. dadurch erfolgen, dass ein Bauteil zur Veränderung der Form und/oder des Umfangs des Grundkörpers, z. B. ein mechanisch oder elektrisch verschiebbarer Keil, vorgesehen ist, wobei grundsätzlich auch andere Mechanismen, wie beispielsweise scherenförmig angeordnete Hebel, etc. zur Verstellung vorgesehen sein können.

Besonders bevorzugt ist dabei vorgesehen, dass der Grundkörper aus mindestens zwei Schalen, vorzugsweise aus drei Schalen aufgebaut ist, die als Spannschalen bezeichnet werden, wobei die Schalen voneinander wegbewegbar sind und so den Umfang des Grundkörpers vergrößern. Diese Bewegung kann vorzugsweise über eine Gewindestange, an der ein Motor stirnseitig angebracht ist, erfolgen.

Die Spannschalen bilden zusammen den Grundkörper und sind dabei jeweils 120° zueinander versetzt angeordnet. Die Aufspreizung kann mechanisch über ein Hebelsystem oder pneumatisch über einen Pneumatikzylinder erfolgen. Auch sind motorisch angetriebenen Lineareinheiten zur Realisierung der Aufspreizung denkbar. Z.B. kann eine Spindel motorisch angetrieben werden, die über eine Mechanik die Aufspreizung erzeugt.

Besonders bevorzugt ist dabei vorgesehen, dass der Grundkörper in der Messvorrichtung horizontal angeordnet ist. Auf diese Weise wird ein Zusammenwirken mit der Wickeleinrichtung erleichtert und insbesondere der Einfluss der Schwerkraft beim Aufwickeln über die Länge des Grundkörpers weitgehend ausgeschaltet.

Darüber hinaus ist der Grundkörper um seine Längsachse drehbar angeordnet, wobei dies insbesondere über einen Flansch erfolgen kann, der mittels eines Motors den Grundkörper antreibt.

Schließlich umfasst die Messvorrichtung eine Wickeleinrichtung zum Aufwickeln von Kompressionsbinden auf den Grundkörper. Hierdurch wird erreicht, dass die Kompressionsbinden standardisiert und normgerecht auf die Messvorrichtung aufgewickelt werden, so dass hierdurch eine vergleichende Beurteilung verschiedener Bandagen unabhängig von dem individuellen Aufwickeln durch eine Person auf die Messvorrichtung möglich ist.

Hierbei ist vorgesehen, dass entweder der Grundkörper oder die Wickeleinrichtung über einen Linearantrieb translatorisch in Längsrichtung des Grundkörpers verfahrbar sind. Besonders ist dabei die Wickeleinrichtung verfahrbar ausgestaltet. Dabei erfolgt ein Aufwickeln einer Binde auf den Grundkörper neben der Linearverschiebung der Wickeleinrichtung auch dadurch, dass sich der Grundkörper um seine eigene Achse dreht bzw. rotatorisch angetrieben ist.

Weiterhin ist die Spannkraft mit der die Binde auf den Grundkörper aufgewickelt werden kann, einstellbar, vorzugsweise regelbar. Hierzu wird ein Ende der Binde am Grundkörper festgelegt und der Rest der Binde in der Wickeleinrichtung geführt, wobei die Spannkraft zwischen Wickeleinrichtung und Grundkörper gemessen wird und auf einen definierten Wert eingestellt werden kann.

Dabei kann vorgesehen sein, dass die Kompressionsbinde in eine Kassette eingelegt wird und drehzahlgeregelt geführt wird, wobei hierfür insbesondere zwei Rollen vorgesehen sind, wovon eine insbesondere drehzahlgeregelt geführt ist. Hierbei kann insbesondere eine Tänzerregelung durchgeführt werden. Hierdurch erfolgt eine Vorspannung der Binde und die Binde wird auf den sich drehenden Grundkörper aufgewickelt.

Besonders bevorzugt kann dabei vorgesehen sein, dass eine Spannkraft von bis zu 150 N geregelt aufgebracht werden kann.

Über den Linearantrieb kann die Steigung und somit die Überlappung beim Aufwickeln der Kompressionsbinde auf den Grundkörper eingestellt werden.

Dabei ist vorgesehen, dass am Grundkörper, sofern die Grundkörper in der bevorzugten Ausgestaltung als Spannschalen ausgebildet sind, an einer oder mehreren der Spannschalen Drucksensoren eingebettet werden also wenigstens teilweise in diese versenkt sind, wobei vorzugsweise auch die Anschlusskabel für die Drucksensoren in einer Vertiefung geführt werden, so dass diese nicht zu einer Erhöhung des Drucks führen.

An dem Grundkörper werden dabei bevorzugt ein oder mehrere, besonders bevorzugt drei Sensoren, in Längsrichtung, d. h. in Richtung der Drehachse, vorzugsweise in einer Linie angebracht.

Diese Sensoren werden dann mit einer Mess- und Auswerteeinrichtung verbunden.

Weiterhin kann vorgesehen sein, auf dem Grundkörper eine diesen zumindest teilweise umschließende Druckmesschicht vorzusehen. Diese kann vorzugsweise als Druckmessfolie ausgebildet sein. Hierüber wird eine vergleichsweise flächige Druckmessung erreicht. Die Druckmessfolie kann dabei so ausgebildet sein, dass eine Messung der Drücke lokaler Bereiche der Druckmessschicht möglich ist. Die Druckmessschicht kann jedoch auch aus einzelnen Sensoren aufgebaut sein oder einzelne Sensoren umfassen, die auf oder in der Druckmessschicht angeordnet sind. Unter Druckmessschicht soll daher im weitesten Sinne lediglich eine fiktive oder tatsächliche Lage verstanden sein, die den Verformungskörper umschließt und in deren Ebene die Druckmesssensoren vorgesehen sind. Dabei sind die Sensoren bevorzugt in eine Materiallage eingebettet also zumindest teilweise in der Lage versenkt angeordnet. So können die Nachteile nicht auftreten, wie sie durch punktuelle Drucksensoren entstehen, die zu Auswölbungen der Kompressionsmaßnahme führen und sich nicht den gekrümmten Gliedmaßen vollständig anzupassen vermögen.

Generell ist es aber auch denkbar die Sensoren in den Grundkörper einzubetten anstatt diese auf der äußeren Fläche des Grundkörpers anzuordnen. Die Sensoren sind dann zumindest teilweise im Grundkörper versenkt angeordnet. Hierdurch wird ebenfalls erreicht, dass die Sensoren nicht zu einer oder nur zu einer geringen Druckerhöhung führen, da sie die Binde nicht dehnen.

Grundsätzlich sind weitere Schichten, die auf dem Grundkörper aufgebracht sind und diesen insbesondere konzentrisch zumindest teilweise umschließen denkbar. So kann z.B. eine oder mehrere Verformungsschichten aus tierischem oder synthetischen Material vorgesehen sein, die Gewebe der Gliedmaße simuliert, vorgesehen sein. Denkbar sind auch mehrere Druckmesschichten sowie eine äußere Lage, die z.B. aus einem Moosgummi oder einer Folie bestehen kann und die Haut simuliert, wobei die Druckmessung dann unterhalb der äußeren Lage in einer Druckmessschicht oder dem Grundkörper stattfindet und die Kompressionsbinde auf die äußere Lage aufgewickelt ist. So kann der Druck in der Tiefe des Gewebes der Gliedmaße abgebildet werden. Ebenfalls denkbar sind Trennlagen zwischen den einzelnen Schichten, die insbesondere bei einer entfernbaren und zu entsorgenden Verformungsschicht vorgesehen sein können und diese einschließen.

Des Weiteren erfolgt eine Messdatenerfassung, wobei auch diese Daten durch die Mess- und Auswerteeinrichtung erfasst werden, insbesondere aus einem Rücklesen von Daten, die für die Regelung der Anlegekraft nötig sind. So kann die Spannkraft der Binde sowie die Drehzahl der Antriebe herangezogen werden. Darüber hinaus kann die Überlappung der Wicklungen der Kompressionsbinde über die Geschwindigkeit des Linearantriebs zurück gemessen werden.

Die Datenübertragung der Drucksensoren kann mit Hilfe von Schleifringen erfolgen. Hierfür wird ein Umsetzer benötigt, der auch die Spannungsversorgung für die Messtechnik mitschleift. Besonders bevorzugt werden die Sensoren z.B an eine Messhardware angeschlossen. Diese setzt die Messdaten in ein USB-Protokoll um und sendet diese an einen PC. Über den Umsetzer erfolgt gegebenenfalls des Weiteren eine Umwandlung der Daten von USB auf Ethernet.

Alternativ ist auch die Übertragung mittels Bluetooth oder eines direkten TCP/IP-Protokolls von den Drucksensoren sowie über eine mögliche serielle Schnittstelle von den Drucksensoren möglich.

Es erfolgt hierbei bevorzugt eine Regelung der Anlegekraft der Binde. Dabei wird die Zugkraft der Binde geregelt. Hierbei wird vorzugsweise das Verfahren einer Tänzerregelung eingesetzt. Dabei wird über die Drehzahl des Grundkörpers und die Drehzahl der Binde eine Kraft eingestellt und auf diese geregelt. Die Kraft kann vorzugsweise zwischen 10 N und 150 N liegen, besonders bevorzugt zwischen 30 N bis 100N.

Die Anlegekraft wird dabei über eine Kraftmessdose bestimmt. Diese weist vorzugsweise eine Genauigkeit von ± 0,02 % zuzüglich Nichtlinearität, Messverstärker und Temperatureinfluss auf. Weitere Einflüsse wie die Streuung der Kompressionsbinden müssen gegebenenfalls berücksichtigt werden.

Je nach Auswahl der Drucksensoren wäre jedoch auch eine direkte Regelung des Anlegedrucks möglich. Hierzu müssten Drucksensoren verwendet werden, die ein kontinuierliches Signal liefern und eine Latenzzeit besitzen hinsichtlich der Datenübertragung, die eine Regelung zulässt.

Die Überlappung der einzelnen Bindentouren beim Aufwickeln auf den Grundkörper erfolgt mit Hilfe eines Linearantriebs einer Lineareinheit. Diese verfährt vorzugsweise die Wickeleinheit parallel zur Längsachse des Grundkörpers während der Bewicklung. Dabei rotiert der Grundkörper. Die Überlappung kann als Prozentangabe bezogen auf die Breite der Binde angegeben werden. Abhängig von der Geschwindigkeit des Grundkörpers und der prozentualen Überlappung kann dann die Geschwindigkeit der Lineareinheit geregelt werden.

Insgesamt können vorzugsweise drei oder sogar mehr Binden übereinander auf dem Grundkörper aufgewickelt werden.

Das Messen mit der Messvorrichtung erfolgt nun insbesondere dergestalt, dass zunächst die zu testende Binde in die Wickeleinrichtung eingelegt wird und durch die beiden Rollen für die Krafteinleitung geführt und über eine Klemmvorrichtung am Grundkörper fixiert wird. Die Binde kann dann vorgespannt werden. Hierbei sollte sichergestellt werden, dass die Kompressionsbinde eine Umdrehung ohne Falten auf dem Grundkörper aufliegt. Dies erfolgt in der Regel außerhalb der nutzbaren Wickellänge.

Mit weiteren Umdrehungen wird die Kraft in der Kompressionsbinde eingestellt. Sobald dies erfolgt ist, kann der eigentliche Wickelablauf gestartet werden. D. h. die Kompressionsbinde wird von der Wickeleinrichtung auf den Grundkörper aufgewickelt. Der Grundkörper dreht sich dabei mit einer konstanten Geschwindigkeit, die vorzugsweise bei 10 bis 15 U/min liegt. Die Wickeleinrichtung bewegt sich dabei in Abhängigkeit von der Bindenbreite, der Überlappung und der Anzahl der Lagen translatorisch mittels einer Lineareinheit. Der Vorgang wird so beendet, dass die Binde noch bis zum letzten Wickeln von den beiden Spannrollen gehalten wird und von Hand am Grundkörper fixiert werden kann.

Sollen mehrere Kompressionsbinden aufgewickelt werden, wird der entsprechende Vorgang wiederholt.

Sobald das Aufwickeln abgeschlossen ist, wird der Umfang des Grundkörper bzw. der äußersten auf dem Grundkörper aufgebrachten Schicht um bis zu 10 mm und insbesondere um genau 10 mm geweitet. Nach der Messdatenerfassung wird der Umfang wiederum reduziert und die Messung ist beendet. Die Kompressionsbinde kann nun manuell entnommen werden.

Besonders bevorzugt dient die Messvorrichtung dabei zur Bestimmung des Static Stiffness Index (SSI), der die Differenz zwischen dem gemessenen Druck im Stehen und im Liegen jeweils in mm Hg : 1 cm angibt. In idealer Weise wird dies im Bereich des Unterschenkels erreicht, wenn dessen Umfang sich um 1 cm vergrößert durch den Positionswechsel zwischen der entspannten liegenden Position (auf dem Rücken) und der aktiven stehenden Position.

Des Weiteren umfasst die Erfindung ein Messverfahren, wobei das Messverfahren die Verwendung der vorstehend beschriebenen Messvorrichtung umfasst, sowie das Anlegen der Kompressionsmaßnahme, insbesondere das Anlegen eines Kompressionsverbandes mittels einer Wickelapparatur. Bei der Messung wird der herrschende Druck P1 mittels der Drucksensoren ermittelt. Nach einer Umfangsänderung des Grundkörpers bzw. der äußersten auf diesem vorgesehenen Schicht um genau 1 cm, die vorzugsweise durch die Änderung der Querschnittsfläche des Grundkörpers erreicht wird, wird dann der herrschende Druck P2 nach der Umfangsänderung wiederum mit Hilfe der Drucksensoren bestimmt. Das resultierende Messergebnis ist dann der sogenannte Static Stiffness Index (SSI) in vitro, der sich aus der Differenz der beiden Drücke P2 und P1 gemäß der Formel SSI(in vitro) = P2 - P1 ergibt

Möglich ist auch eine Bestimmung des Dynamic Stiffness Index (DSI) durch eine dynamische Messung, z.B. mittels periodischer/oszillierender Aufweitungen bzw. Zufahren des Grundkörpers.

Weitere Vorteile und Merkmale der Erfindung ergeben sich aus den übrigen Anmeldungsunterlagen.

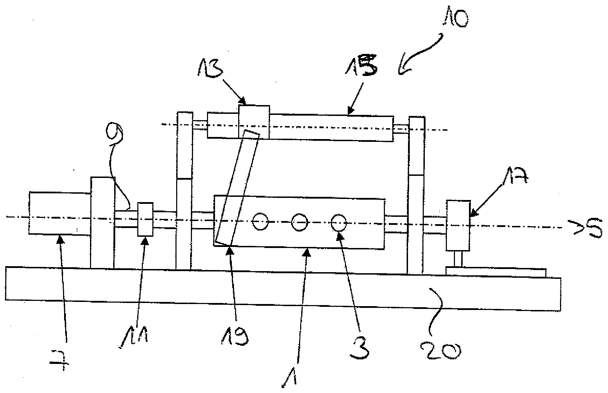

Die Erfindung wird im Folgenden anhand einer Zeichnung näher erläutert. Dabei zeigt die einzige Figur eine schematische Darstellung der Meßvorrichtung.

Die Erfindung betrifft eine Messvorrichtung, die in ihrer Gesamtheit mit dem Bezugszeichen 10 versehen ist. Die Vorrichtung umfasst dabei eine Standplatte 20, auf der die Vorrichtung montiert ist. Des Weiteren umfasst die Vorrichtung 10 zum Bestimmen des Kompressionsdrucks von Kompressionsbinden einen um seine Längsachse drehbaren Grundkörper 1, der als Beinnachbildung fungiert und aus drei Schalen (nicht dargestellt) ausgebildet ist, die im 120°-Winkel zueinander angeordnet sind, wobei die Schalen über eine Mechanik aufspreizbar sind, so dass sich der Umfang des Grundkörpers 1 verändert.

Auf einer der Schalen des Grundkörpers 1, der die Beinnachbildung bildet, sind drei Sensoren 3 angeordnet, wobei die Sensoren in Längsrichtung des Grundkörpers 1, die mit dem Pfeil 5 gekennzeichnet ist, hintereinander und beabstandet angeordnet sind, wobei die Sensoren 3 im Wesentlichen gleichmäßig über die Länge des Grundkörpers 1 angeordnet sind. Zum Antrieb des Grundkörpers 1 ist dabei ein Motor 7 vorgesehen, der über eine Welle 9, die mit dem Motor über eine Kupplung 11 verbunden ist, den Grundkörper 1 drehend antreibt.

Darüber hinaus ist eine Wickeleinrichtung 13 vorgesehen, die auf einer Lineareinheit 15 translatorisch verschiebbar geführt ist, wobei die Wickeleinrichtung 13 achsparallel zur Längsachse 5 des Grundkörpers 1 angeordnet ist und die Lineareinheit 15 im Wesentlichen so angeordnet ist, dass die Wickeleinheit 13 über die gesamte Länge des Grundkörpers 1 translatorisch verschoben werden kann. Darüber hinaus ist dann mit der Welle noch eine Einrichtung zur Signalübertragung 17 verbunden, die die über die Sensoren 3 aufgenommenen Daten an eine nicht gezeigte Mess- und Auswerteeinrichtung überträgt.

Die Wickeleinrichtung 13 umfasst dabei eine Aufnahme für eine Binde 19 sowie Spannrollen. Die Binde 19 wird außerhalb der eigentlichen Messzone mit dem Grundkörper 1 verbunden und vorgespannt. Die Kraft wird über einen Sensor (nicht dargestellt) eingestellt. Es erfolgt dann außerhalb der eigentlichen Messzone eine oder mehrere Umwicklungen der Kompressionsbinde 19 auf den Grundkörper 1 zum Einstellen der Kraft. Sobald diese auf den gewünschten Wert eingestellt wurde, kann die Binde 19 automatisiert aufgewickelt werden, wobei über die Lineareinrichtung 15 die Art der Aufwicklung gesteuert wird. Hierbei sind insbesondere die Bindenbreite, aber auch die Überlappung relevante Parameter. Das Ende der Binde 19 wird manuell am Grundkörper 1 festgelegt.

Wenn die Binde 19 dann auf den Grundkörper 1 aufgewickelt ist, erfolgt zunächst eine Druckmessung über die Sensoren 3, die das Signal über die Signalübertragungseinrichtung 17 auf die Mess- und Auswerteeinrichtung übertragen. Danach wird der Grundkörper 1 so aufgespreizt, dass der Umfang des Grundkörpers oder der äußersten auf dem Grundkörper vorgesehenen Schicht sich um 1 cm vergrößert. Es wird dann eine erneute Messung vorgenommen. Auf diese Weise kann der Static Stiffness Index bestimmt werden. Nach Messung kann die Binde 19 dann manuell entfernt werden.

Der besondere Vorteil der Vorrichtung 10 liegt in der Möglichkeit der Messung des Static Stiffness Index (SSI) bzw. des DSI in einem in vitro-Verfahren mit reproduzierbaren und standardisierten Bedingungen, die gleichwohl jedoch eine bestmögliche Anpassung an die Gegebenheiten an einer menschlichen oder tierischen Gliedmaße ermöglichen.

## Patentansprüche

1. Messvorrichtung (10) zum Bestimmen des Kompressionsdrucks von Kompressionsbinden (19) umfassend einen um seine Längsachse (5) drehbaren Grundkörper (1) geeignet für das Bewickeln mit einer Kompressionsbinde (19) sowie eine Wickeleinrichtung (13) geeignet für die Aufnahme und Abgabe der Kompressionsbinde (19) an den Grundkörper (1), wobei der Grundkörper (1) und/oder die Wickeleinrichtung (13) über eine Lineareinheit (15) translatorisch bewegbar ist und der Grundkörper (1) Sensoren (3) zum Messen des Kompressionsdrucks von Kompressionsbinden (19) aufweist, die mit einer Mess- und Auswerteeinrichtung verbunden sind.

2. Messvorrichtung (10) nach Anspruch 1, wobei der Grundkörper (1) eine zylindrische oder kegelstumpfförmige Grundform aufweist.

3. Messvorrichtung (10) nach einem der vorangehenden Ansprüche, wobei der Querschnitt des Grundkörpers (1) rund oder oval ist.

4. Messvorrichtung (10) nach den Ansprüchen 2 und 3, wobei sowohl die Grundform als auch die Querschnittsform über die Länge des Grundkörpers (1) variabel ist.

5. Messvorrichtung (10) nach einem der vorangehenden Ansprüche, wobei der Grundkörper (1) eine einstellbare Querschnittsform und/oder einen einstellbaren Umfang aufweist, wobei der Grundkörper (1) eine mittels einer Verstellvorrichtung (2) einstellbare Querschnittsform und/oder einen einstellbaren Umfang aufweist.

6. Messvorrichtung (10) nach einem der vorangehenden Ansprüche, wobei der Grundkörper aus mindestens drei Schalen besteht, die mit der Verstellvorrichtung verbunden sind.

7. Messvorrichtung (10) nach einem der vorangehenden Ansprüche, wobei der Grundkörper (1) zumindest teilweise durch eine Druckmessschicht umschlossen ist, die die Sensoren (3) umfasst.

8. Messvorrichtung (10) nach einem der vorangehenden Ansprüche, wobei die Druckmesschicht als Druckmessfolie ausgebildet ist.

9. Messvorrichtung (10) nach einem der vorangehenden Ansprüche, wobei die Sensoren (3) in den Grundkörper (1)
oder die Druckmessschicht eingebettet sind
und zumindest teilweise in dem Grundkörper (1) oder der Druckmessschicht versenkt angeordnet sind.

10. Messvorrichtung (10) nach einem der vorangehenden Ansprüche, wobei der Grundkörper (1) mittels eines Motors (7) drehend antreibbar ist.

11. Messvorrichtung (10)
nach einem der vorangehenden Ansprüche zur Bestimmung
des Static Stiffness Index (SSI) oder des Dynamic Stiffness Index (DSI).

## Claims

1. A measuring device (10) for determining the compression pressure of compression dressings (19), including a base body (1) which can be rotated about its longitudinal axis (5) and is suitable for being wrapped with a compression dressing (19) as well as a wrapping device (13) suitable for receiving the compression dressing (19) and dispensing it to the base body (1), wherein the base body (1) and/or the wrapping device (13) is movable translationally via a linear unit (15), and the base body (1) has sensors (3) for measuring the compression pressure of compression dressings (19), which sensors are connected to a measuring and evaluation device.

2. The measuring device (10) of claim 1, wherein the base body (1) has a cylindrical or frustoconical basic shape.

3. The measuring device (10) of one of the foregoing claims, wherein the cross section of the base body (1) is round or oval.

4. The measuring device (10) of claims 2 and 3, wherein both the basic shape and the cross-sectional shape are variable over the length of the base body (1).

5. The measuring device (10) of one of the foregoing claims, wherein the base body (1) has an adjustable cross-sectional shape and/or an adjustable circumference, and the base body (1) has a cross-sectional form adjustable and/or has an adjustable circumference by means of an adjusting device (2).

6. The measuring device (10) of one of the foregoing claims, wherein the base body consists of at least three shells, which are connected to the adjusting device.

7. The measuring device (10) of one of the foregoing claims, wherein the base body (1) is surrounded at least partially by a pressure measurement layer, which includes the sensors (3).

8. The measuring device (10) of one of the foregoing claims, wherein the pressure measurement layer is embodied as a pressure measurement film.

9. The measuring device (10) of one of the foregoing claims, wherein the sensors (3) are embedded in the base body (1) or in the pressure measurement layer, and are located at least partially sunken in the base body (1) or the pressure measurement layer.

10. The measuring device (10) of one of the foregoing claims, wherein the base body (1) can be driven to rotate by means of a motor (7).

11. The measuring device (10) of one of the foregoing claims for determining the static stiffness index (SSI) or the dynamic stiffness index (DSI).

## Revendications

1. Dispositif de mesure (10) destiné à déterminer la pression de compression de bandages compressifs (19), comprenant un corps de base (1), pouvant être amené en rotation autour de son axe longitudinal (5), adapté à l'enroulement avec un bandage compressif (19) ainsi qu'un dispositif d'enroulement (13) adapté à la réception et distribution du bandage compressif (19) sur le corps de base (1), dans lequel le corps de base (1) et/ou le dispositif d'enroulement (13) peuvent être déplacés en translation par l'intermédiaire d'une unité linéaire (15) et le corps de base (1) présente des capteurs (3) destinés à mesurer la pression de compression de bandages compressifs (19), qui sont reliés à un dispositif de mesure et d'évaluation.

2. Dispositif de mesure (10) selon la revendication 1, dans lequel le corps de base (1) présente une forme de base cylindrique ou tronconique.

3. Dispositif de mesure (10) selon l'une quelconque des revendications précédentes, dans lequel la section transversale du corps de base (1) est ronde ou ovale.

4. Dispositif de mesure (10) selon les revendications 2 et 3, dans lequel aussi bien la forme de base que la forme de section transversale est variable sur toute la longueur du corps de base (1).

5. Dispositif de mesure (10) selon l'une quelconque des revendications précédentes, dans lequel le corps de base (1) présente une forme de section transversale réglable et/ou une périphérie réglable, dans lequel le corps de base (1) présente une forme de section transversale réglable au moyen d'un dispositif de réglage (2) et/ou une périphérie réglable.

6. Dispositif de mesure (10) selon l'une quelconque des revendications précédentes, dans lequel le corps de base est constitué d'au moins trois coques, qui sont reliées au dispositif de réglage.

7. Dispositif de mesure (10) selon l'une quelconque des revendications précédentes, dans lequel le corps de base (1) est entouré au moins en partie par une couche de mesure de pression, qui comprend les capteurs (3).

8. Dispositif de mesure (10) selon l'une quelconque des revendications précédentes, dans lequel la couche de mesure de pression est réalisée en tant que feuille de mesure de pression.

9. Dispositif de mesure (10) selon l'une quelconque des revendications précédentes, dans lequel les capteurs (3) sont intégrés dans le corps de base (1) ou la couche de mesure de pression et sont disposés de manière au moins en partie enfoncée dans le corps de base (1) ou la couche de mesure de pression.

10. Dispositif de mesure (10) selon l'une quelconque des revendications précédentes, dans lequel le corps de base (1) peut être entraîné en rotation au moyen d'un moteur (7) .

11. Dispositif de mesure (10) selon l'une quelconque des revendications précédentes destiné à déterminer l'indice de rigidité statique (SSI) ou l'indice de rigidité dynamique (DSI).
